(19) Europäisches Patentamt
European Patent Office
Office européen des brevets

(11) **EP 2 185 211 B1**

(12) **EUROPEAN PATENT SPECIFICATION**

(45) Date of publication and mention of the grant of the patent:
**27.03.2013 Bulletin 2013/13**

(21) Application number: **08780350.8**

(22) Date of filing: **01.08.2008**

(51) Int Cl.:
*A61L 31/04* (2006.01)          *A61L 31/14* (2006.01)

(86) International application number:
**PCT/US2008/009291**

(87) International publication number:
**WO 2009/020557 (12.02.2009 Gazette 2009/07)**

(54) **KNIT PTFE ARTICLES AND MESH**

GEWIRKTE PTFE-ARTIKEL UND MASCHENGEFLECHT

ARTICLES DE PTFE TRICOTÉS ET MAILLE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.08.2007 US 833566**

(43) Date of publication of application:
**19.05.2010 Bulletin 2010/20**

(73) Proprietor: **Gore Enterprise Holdings, Inc. Newark, DE 19714-9206 (US)**

(72) Inventors:
• **ROEBER, Peter, J.**
  **Wallingford, PA 19086 (US)**
• **BAILEY, Eileen, M.**
  **Landenberg, PA 19350 (US)**
• **MANN, James, W.**
  **Elkton, MD 21921 (US)**
• **NARAYAN, Anand**
  **Bear, DE 19701 (US)**

(74) Representative: **Shanks, Andrew et al**
  **Marks & Clerk LLP**
  **Aurora**
  **120 Bothwell Street**
  **Glasgow**
  **G2 7JS (GB)**

(56) References cited:
WO-A-2005/094741     US-A- 3 054 406
US-A- 5 645 915     US-A1- 2005 222 591

**Description**

BACKGROUND OF THE INVENTION

Mesh fabric prostheses are used in various surgical procedures including repair of anatomical defects of abdominal wall, diaphragm, and chest wall, correction of defects in the genitourinary system, and repair of traumatically
damaged organs such as the spleen, liver, or kidney. Hernia repairs are among the more common surgical operations
that employ such prostheses.

Surgical repair mesh fabrics are constructed from a variety of synthetic fibers in the form of knitted and woven
fabrics.

Ventral hernias can be repaired using open or laparoscopic techniques that include intraperitoneal or preperitoneal placement of a prosthetic biomaterial (e.g. mesh or patch). In the US it is estimated that about 16% (US Markets
for Soft Tissue Repair 2006, Millennium Research Group) of ventral hernias are repaired using an open preperitoneal
technique while in Europe the percentage is much higher, approximately 69% (European Markets for Soft Tissue Repair
2006, Millennium Research Group). In these cases, open knit meshes of polypropylene comprise the material of choice,
providing high in-growth, the ability to treat if infected, and sufficient initial stiffness to enable ease of use during implantation of the prosthetic. However, it is reported in the literature that meshes constructed from polypropylene elicit a
prolonged inflammatory response (Klosterhalfen et al., Expert Rev Med Devices (2005); Jan 2(1): 103-17) (Klinge et al.,
Eur J Surg (1999); 165: 665-673) or chronic foreign body response (FBR). This tissue response may lead to potentially
serious long-term complications such as mesh erosion, mesh migration, fistulas, aggressive adhesions when in contact
with the viscera and a reduction in postoperative compliance leading to patient discomfort. Studies within the literature
have evaluated methods to reduce these responses. The solution most commonly pursued is the use of monofilament,
large pore, reduced material polypropylene meshes. These meshes present a reduced surface area for biological interaction thereby reducing the foreign body response. An improved solution is through substitution of polypropylene with
a more biocompatible material such as polytetrafluoroethylene (PTFE). PTFE knit mesh currently exists in the market,
for example one such brand is Bard® PTFE Mesh; however, it lacks the appropriate monofilament, reduced material,
large pore structure. Even so, due to the inherently lower modulus of the material, PTFE knit articles have inferior handling
(stiffness), compared to the preferred characteristics of polypropylene mesh. Conventional methods of raising the stiffness
of the knit article result in increased foreign body and or the introduction of an additional material that takes away from
the biocompatibility of the construct. The ideal PTFE knit prosthetic mesh should be constructed such that it combines
the ideal structure and material without sacrificing the desired handling. The present invention addresses this limitation
and enables the creation of a highly biocompatible, monofilament, reduced material, large pore prosthetic mesh with
appropriate handling for the reconstruction of hernias and other soft tissue deficiencies.

US 2005/0222591 (Gingras) discloses a manner in which a mesh for surgical implant can be subsequently
condensed in order to reduce the void spaces between adjacent fibres and reduce the surface area of the fibres for
contact with tissue.

SUMMARY OF THE INVENTION

In one embodiment of the invention is provided a knitted article with a knitted structure having at least one
PTFE fiber with oriented fibrils forming multiple fiber cross-over points wherein the PTFE fiber is self-bonded in at least
one of the cross-over points. The fibrils in the knitted structure are able to self-bond while oriented essentially non-parallel
to each other. In another embodiment the PTFE fiber is a monofilament PTFE fiber.

DESCRIPTION OF DRAWINGS

Figures 1A-1B show an electron microscopy view which illustrates a PTFE knitted article before heat treatment.
Figures 1C-1D illustrate PTFE knitted articles after heat treatment.
Figure 2 is a diagram of various knitted mesh examples of shapes and geometries for the PTFE knitted article.
Figures 3A-3E show a cross-section diagram of a PTFE knitted article used in combination with layered absorbable,
non-absorbable, or biological cells.
Figure 4 shows a ball and mesh as used in the abdominal mesh test of Example 7.
Figure 5 shows a graph describing the BB measurements for three different knit samples as described in Example 5.

DETAILED DESCRIPTION OF THE INVENTION

A knitted PTFE surgical mesh is provided which exhibits both a desirable material and unique handling features, without the need of a bonding additive. The unique ability of the PTFE knit to form articles without the addition of bonding additives, to achieve tissue integration, favorable anti-inflammation results, and good biomechanical resistance for soft tissue repair is an unexpected result derived from the present invention.

A knitted article is constructed of a yarn having at least one PTFE fiber. The term PTFE is meant to be inclusive of expanded polytetra-fluorethylene (ePTFE). The PTFE fiber comprises oriented fibrils. The PTFE fiber may be microporous or non-microporous. In one aspect the PTFE fiber 4 is a monofilament PTFE fiber. In another aspect, the PTFE fiber may be at least two different PTFE fibers having differing deniers, density, lengths or dimensional differences. In another aspect of the present invention, a multiple strand yarn which is comprised of at least one PTFE fiber and at least one other type of fiber that is not PTFE may be knitted. In this aspect, the PTFE fiber in the multiple strand yarn self-bonds at the crossover points of PTFE fiber crossed over PTFE fiber. The PTFE fiber may be the same strand or a differing strand in the same multiple strand yarn. A self bonding at a cross-over point may be created without an adhesive using self-bonding techniques.

A non-microporous PTFE fiber prevents the penetration and harboring of bacteria. Open knitted structures provide for tissue in-growth and may provide for better infection treatable implants. A PTFE knit is a monofilament structure in its simplest form. The structure may be filled or loaded with therapeutic agents, to facilitate, for example, drug delivery, as desired.

The PTFE fiber is knitted into an article, forming multiple fiber cross-over points formed where the PTFE fiber is in contact with itself.
The PTFE yarn is configured into the desired knit pattern and the knit pattern is then heat treated to increase stiffness. The heating bonds the PTFE fiber onto itself in at least one cross-over point.

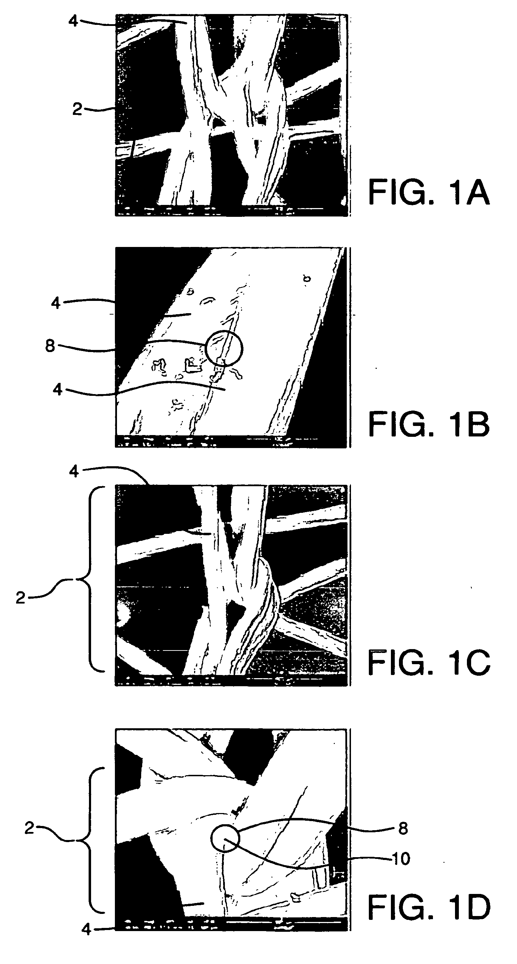

Figure 1A shows an example of the structure of a PTFE knitted article prior to heat treatment. A PTFE fiber 4 is formed into a knitted structure 2 so that the PTFE fiber 4 forms multiple fiber crossover points 8 wherein the PTFE fiber 4 has a self-bonded region 10 in at least one of the cross-over points 8. Figure 1B shows a crossover point 8 of two PTFE fibers 4 in a knitted structure wherein no self-bond regions occur as illustrated by no evidence of bonding between the areas of contact allowing the fibers to move independent of each other.

One way to form the knitted structure 2 of the present invention is by configuring the PTFE fiber 4 into a knit pattern formed onto or attached to a restraining means 12, and exposing the knitted structure 2 to heat while the knitted structure 2 remains fully constrained, thus preventing the knitted structure from contracting or moving. The restraining means 12 may be a pin hoop, clasp, frame, or any suitable mechanism which allows the knit to be firmly affixed and heated. When exposed to heat, the PTFE fiber 4 will shrink in a primarily longitudinal manner. This shrinkage causes the constrained article on the pin hoop to become taut which creates pressure at the cross-over points or the intersections of the fibers within the knitted article.

It was unexpected that PTFE fibers self-bond without externally applied pressure and heat or the use of a bonding agent. This type of bonding is an advantage in producing a fully PTFE or fully ePTFE knit article. The temperature at which the fibrils are able to self-bond ranges from between 327-400°C. A controlled temperature of between 350-370°C and a controlled exposure time for a period of about 5-10 minutes may be desired in the processing of meshes.

The stiffness of the knit may be increased with longer durations of heating at a controlled temperature setting. The self-bonding of two or more PTFE fibers with oriented fibrils is a result of being contacted under heat and fiber to fiber tension, allowing the fibers to fuse or lock at the interfaces of the fibers without the need of a bonding agent. When the fibrils are fused together, they are prevented from relative movement with regard to each other, referred to as cross-fibril locking. The knitted structure 2 may be cooled prior to removing from the restraining means. The cooled article may then be cut into a finished geometry using various methods known in the art. The bonding at the multitude of fiber intersections within the textile results in a significant increase in the stiffness of the article providing more preferred handling characteristics. For instance, if desired, the resulting stiffness of a knit structure can be increased by at least 50%, over the same knit structure in a non-self-bonded form. Accordingly, various knit patterns and fiber deniers may result in differing stiffness measurements.

The stiffness of PTFE knitted structures may be modulated as desired by choice of material thickness and composition, and measured by standard tests, i.e. INDA Standard Test IST 90.3 (95) Handle-O-Meter of Nonwoven Fabrics. For instance, a PTFE knitted structure in the form of a single thickness mesh may be formed having a mass between 50-70 g/m$^2$, 70-90 g/m$^2$, or 90-165 g/m$^2$ and having a stiffness of greater than 25g, 35g, or 50g, respectively.

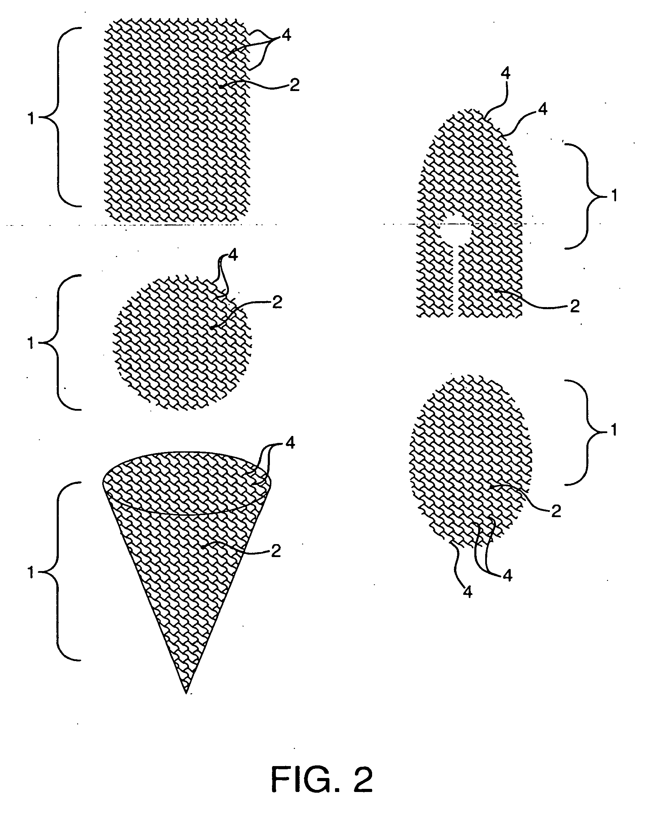

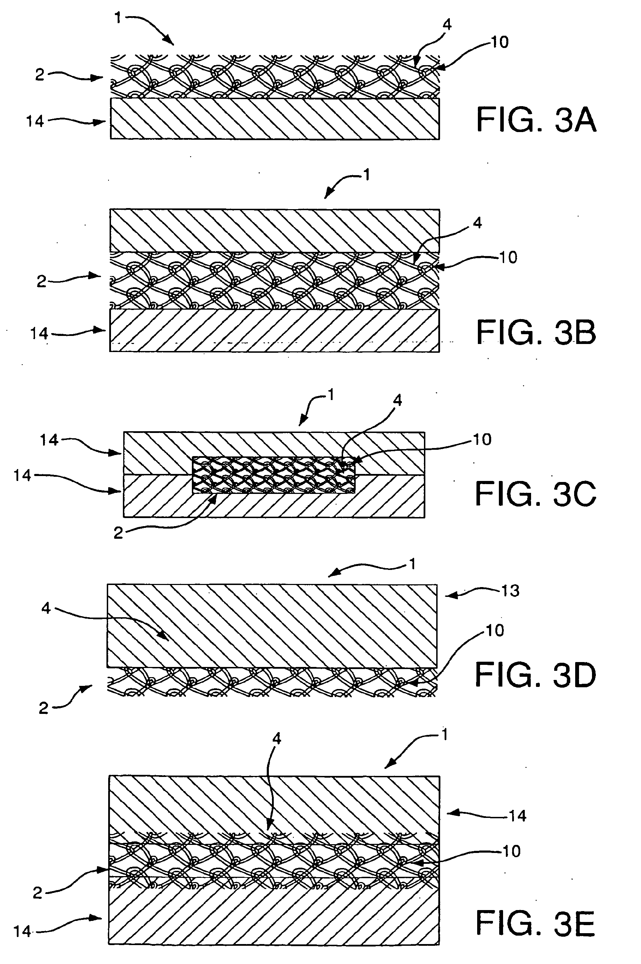

Figure 2 shows diagrams of articles 1 having knitted structures 2 formed of PTFE fiber 4. The knitted structure in these embodiments is solely in the form of a knitted mesh of various shapes and geometry. As shown in Figures 3A-3E below, the knitted structure may be combined with other elements to form desired articles.

A surprising feature of the knitted structure 2 is that the PTFE fiber 4 comprises oriented fibrils 6 which may be self-bonded in a relatively non-parallel configuration which allows the structure to have increased pattern stability and stiffness.

A PTFE knit structure of the present invention is able to be easily handled due to the self-bonded regions, hence providing additional stability and stiffness to prevent twisting and wrinkling associated with traditional PTFE articles. Similarly, articles formed of the present invention may provide a patient with a more comfortable article which provides support to the injured area and also provides the patient with greater flexibility of the *in situ* article.

As exemplified in Figures 3A-3E, the PTFE knitted structure 2 may be fabricated into articles having at least one knitted structure and at least one other material to form a composite structure. Figure 3A shows an article 1 formed of a PTFE knitted structure 2 and a functional layer 14. The knitted structure comprises at least one PTFE fiber 4 formed into the knitted structure 2 so that the PTFE fiber 4 forms multiple fiber cross-over points wherein the PTFE fiber 4 has a self-bonded region 10 in at least one of the cross-over points. The functional layer 14 may comprise either a non-absorbable, absorbable, or resorbable material. Examples of non-absorbable materials include but are not limited to ePTFE, polypropylene, polyester, and fluoropolymers. Examples of absorbable materials include but are not limited to polymers or co-polymers or blends of absorbable materials. The knitted structure 2 and a functional layer 14, as illustrated in Figures 3A-3C may be laminated or joined by various methods known in the art or as described in the examples below.

Figure 3B shows an article having a composite structure formed of a PTFE knitted structure 2, and two functional layers 14. In this figure, one layer comprising a PTFE knitted structure 2 having a top side and a bottom side is joined to a functional layer 14 on its top side and a functional layer 14 on its bottom side forming an article. As discussed above, the functional layer 14 may be absorbable or non-absorbable or a combination of absorbable and non-absorbable material. It is also within the scope of the invention that more than one knitted structure may be present in a single article. Further, it is similarly within the scope of the invention that more than two functional layers may be present in a single article. The article may be used in various manners including as a surgical material, surgical mesh, hernia repair, soft tissue repair, soft tissue reinforcement, repair of anatomical defects, correction of defects in the genitourinary system, repair of traumatically damaged organs including spleen, liver, kidney, or any combination thereof, anatomical defect repair including defects in the abdominal wall, diaphragmatic, chest wall, or any suitable use or combination thereof.

Figure 3C shows the PTFE knitted structure 2 fabricated into an article such that the knitted structure is fully enclosed between two functional layers 14 to form a composite structure. The functional layers may be comprised of similar or different materials, and may also each have similar or different properties, such as rate of absorption, drug eluting properties, or mechanical properties such as stiffness. The functional layers may be joined or bonded together as discussed above including via lamination, an absorbable adhesive, or other means of joining.

The PTFE knitted structure 2 can further be gravure printed with a discrete pattern of adhesive. The pattern can be designed such that it closely matches that of the knit pattern allowing maximum surface coverage with absorbable adhesive. The knitted structure 2, with adhesive present, can then be laminated to a non-woven self adhering web. Other methods for manufacturing the composite article include but are not limited to thermal bonding, sewing, solvent bonding, using a tie layer of a separate absorbable material to join layers or permanent layer coatings or similar known means for forming composite articles. One skilled in the art could use these processes to achieve a multi-layer composite article of at least one PTFE knitted structure 2 and at least one functional layer.

Other examples of articles of the present invention include PTFE knit structures formed into surgical meshes and incorporated into multi-layer composite constructions having individual absorbable and non-absorbable layers imbibed throughout at least a portion of the PTFE knitted structure 2, as illustrated in Figures 3D or 3E.

Figure 3D shows an article 1 comprising a PTFE knitted structure 2 joined with a biological material 13. The biological material is a modulating substance which either enhances wound healing, provides induced cellular or tissue growth of the host to which it contacts or provides induced cellular or tissue growth of the biological material itself.

Figure 3E shows an article having a composite structure formed of a PTFE knitted structure 2, and two functional layers 14. In this figure, one layer comprising a PTFE knitted structure 2 with a self-bonded region 10, and having a top side and a bottom side is joined to a functional layer 14 on its top side and a functional layer 14 on its bottom side forming an article. The functional layer 14 may be imbibed into the knitted structure as shown. It is anticipated that the functional layers could be either partially or fully imbibed into the knitted structure. The functional layer 14 may be absorbable or non-absorbable or a combination of absorbable and non-absorbable material. It is also within the scope of the invention that more than one knitted structure may be present in a single article.

In one embodiment, the knitted article or mesh comprises a knitted structure having at least one PTFE fiber with oriented fibrils. The PTFE fiber forms multiple fiber cross-over points wherein said PTFE fiber is self-bonded (bonded onto itself at the cross-over point of the fiber to form a bond without the use of an adhesive) in at least one cross-over point. The PTFE fiber is a porous structure with an initial stiffness and a second stiffness. The second stiffness is less than the initial stiffness. The initial stiffness decreases to reach a second stiffness when the porous structure is subjected to surface tension. It is desirable that the knitted article soften or decrease stiffness under a tension force equal or less than the typical abdominal muscle tension exerted by a person when an individual is sitting, standing, coughing, or jumping. The softening or decrease of stiffness under a tensional force occurs when a bond in at least one of said cross-over points is broken when the knitted structure is subjected to a surface tension equal or less than intraabdominal pressure, as defined in examples below. The fiber may be knitted, woven or braided.

The PTFE surgical mesh may be coated with absorbable or drug-eluting compositions as described in the examples below or coating methods known in the art. Additionally, it is within scope of the present invention that a bioactive agent, antimicrobial agents, and/or antibiotics may be embedded in the drug-eluting compositions prior to coating the PTFE surgical mesh. The bioactive may be an analgesic, non-steroidal-anti-inflammatory drug (NSAID), or an anesthetic.

## Examples

Example 1

Table 2 illustrates a typical example of knitted mesh as utilized by this invention. These examples are illustrative only and are not intended to limit the scope of the present invention. Various knit patterns may be used to form the PTFE knits used in the present invention.

Table 2

| Knit Pattern Examples | A | B | C | D | E | F |
|---|---|---|---|---|---|---|
| Filament | Mono | Mono | Mono | Mono | Mono | Mono |
| Knit Type | Dual Bar | Dual Bar | Dual Bar | Dual Bar | Dual Bar | Dual Bar |
| WPI [wales per inch] | 19 | 19 | 19 | 18 | 16 | 19 |
| CPI [Courses per Inch] | 24 | 38 | 16 | 21 | 23 | 29 |
| PTFE Fiber [denier] | 195 | 195 | 195 | 195 | 195 | 195 |
| Mesh Weight [g/m2] | 63.4 | 89.5 | 123.3 | 135.5 | 163.8 | 164.4 |
| Pore Size (approx.) [μm] | 2600 | 1700 | 825 | 700 | 2100 | 425 |

Example 2 - Stiffness Measurement

The overall hand, or average textile stiffness, was measured for each of the knit examples in Table 1 according to INDA Standard Test IST 90.3 (95) Handle-O-Meter Stiffness of Nonwoven Fabrics. A summary table of original stiffness can be found below in Table 3. As seen by Table 2 and Table 3, various knit patterns and fiber deniers may result in differing stiffness measurements.

Example 3 - Stiffening Treatment

The untreated PTFE knits exemplified in Table 2 were treated to increase stiffness by attaching the textile to a pin hoop such that when exposed to heat the material will be fully constrained, thus preventing the article from contracting. The pin hoop is constructed of stainless steel, has a diameter of approximately 24 inches, and has 0.020 inch pin needles approximately 1 inch in length that are spaced evenly every 1-2 inches around the perimeter. The pin hoop, with textile firmly affixed, was then heated to 365°C for 7 minutes. This treatment results in self-bonding at the fiber cross-over points.

Example 4

The article described in example 3 was cooled to room temperature, removed from the pin hoop. The cooled article was cut into a finished geometry. The bonding at the multitude of fiber intersections (self-bonds) within the textile results in a significant increase in the stiffness of the article providing more preferred handling characteristics. Table 3 discloses the stiffness measurements of PTFE knits disclosed in Table 2 before and after self-bonding at the fiber cross-over points.

Table 3

| Knit Examples | A | B | C | D | E | F |
|---|---|---|---|---|---|---|
| Mesh Weight [g/m2] | 63 | 89 | 123 | 135 | 164 | 164 |

(continued)

| Knit Examples | A | B | C | D | E | F |
|---|---|---|---|---|---|---|
| Initial Stiffness (g) | 13 | 13 | 21 | 36 | 36 | 41 |
| Final Stiffness (g) | 28 | 46 | 180 | 155 | 415 | 308 |
| Percent Increase (%) | 221% | 362% | 860% | 430% | 1144% | 760% |

Although several examples are described here, one skilled in the art may easily influence mesh mass by changing stitch pattern, fiber denier, or fiber density to influence final mesh stiffness. One skilled in the art may also use different constraining and thermal treatment methods to obtain similar results.

Example 5 - Stiffness change upon deformation

A knit sample was made with the following characteristics:

Knit Pattern: Hexagonal
Knit Type: Dual Bar
Filament: Mono
WPI: 28 +/- 5
CPI: 44 +/- 5
PTFE fiber diameter: <= 127um
Mesh Weight: 2.8 +/- 0.4 oz/yd$^2$
Pore Size > 1000 microns
Experimental Temperature: Room Temperature

The above sample was heat treated at 365°C for 3 minutes.
Multiple samples, 4" X 4" in size, were cut from the above heat treated knit.
Each sample was subjected to the following:

The sample was loaded into the jaws of an Instron (Model # 5564) Universal Testing Machine, such that the direction of strain was the machine direction of the knit. The sample was displaced to a distance of 9 mm, at a rate of 80 mm/min. The load vs. displacement data was acquired. From this data the modulus of the knit along the machine direction was obtained, referred to as first modulus. The modulus is defined as the slope of the Load vs. Displacement Curve. The sample was then removed, and turned 90° such that the direction of strain was now the cross web direction of the knit. The sample was subjected to a displacement of 9 mm at 80 mm/min rate. Upon completion the sample was turned 90° so that the direction of the pull was once again along the machine direction. The load vs. displacement data was again acquired, using the same 80 mm/min rate and a maximum displacement of 9 mm. The modulus of the knit in the machine direction thus obtained for the second time is referred to as the second modulus.

Seventeen samples were measured. The second modulus was lower than the first modulus for each sample tested. The average reduction in the modulus was 47.8% with a minimum change of 26.1 % and a maximum change of 64.5%. The self-bonded knit lost a significant part of its stiffness, as represented by the change in modulus, upon being stretched to a maximum strain of approximately 10%.

Example 6 - Abdominal Mesh Tension Calculation

The values for Mean Intraabdominal Pressure (IAP) were extracted from Cobb *et al.* (see Cobb et al. "Normal Intraabdominal Pressure in Healthy Adults." J Surg Res. 2005 Dec; 129(2):231-5. Epub 2005 Sep 2) and used as representative pressure measurements to calculate Abdominal Mesh Tension. The Abdominal Mesh Tension is considered to be the tension exerted on an implanted knitted mesh or knitted article when the implanted mesh or article is used to repair a defect in the abdominal wall of an individual. The measurement is representative of the tension applied to the knitted mesh or article when the individual is undergoing light activity such as sitting, standing, coughing, or jumping.
The method for calculating Abdominal Mesh Tension (see Table 4 below) is described by Klinge, *et al.* (see Klinge et al. "Modified mesh for hernia repair that is adapted to the Physiology of the abdominal wall." EurJ Surg. 1998 Dec; 164(12):951-60):

$$\text{Mesh Tension}_{hoop} \text{ N/cm} = PD/2$$

$$\text{Mesh Tension}_{longitude} \text{ N/cm} = PD/4$$

Where:

P= Pressure (N/cm)
D=Diameter (32 cm)

Table 4

| Activity | Mean IAP | | Abdominal Mesh Tension (longitude) | Abdominal Mesh Tension (hoop) |
|---|---|---|---|---|
| | mmHg | N/cm$^2$ | N/cm | N/cm |
| Supine | 1.8 | 0.024 | 0.192 | 0.384 |
| Stranding | 20.0 | 0.267 | 2.136 | 4.272 |
| Sitting | 19.6 | 0.231 | 1.848 | 3.696 |
| Stairs | 68.9 | 0.919 | 7.352 | 14.704 |
| Bending at waist | 14.4 | 0.192 | 1.536 | 3.072 |
| Bending at knees | 20.6 | 0.275 | 2.2 | 4.4 |
| Light activity | | | | |
| Min | 1.8 | 0.024 | 0.192 | 0.384 |
| Max | 68.9 | 0.919 | 7.352 | 14.70 |

Example 7 - Abdominal Mesh Tension Test

Load vs. Displacement data was obtained from a modified ASTM D3787-01 Standard Test Method for Bursting Strength of Textiles - Constant-Rate-of-Traverse (CRT) Ball Burst Test. The ASTM test was modified by changing The-Constant-Rate-of-Transverse (CRT) from 300 mm/min to 50 mm/min and also changing the diameter of the steel ball used in the test from 25 mm to 38 mm (The modified test is referred to as "BB" hereinafter).

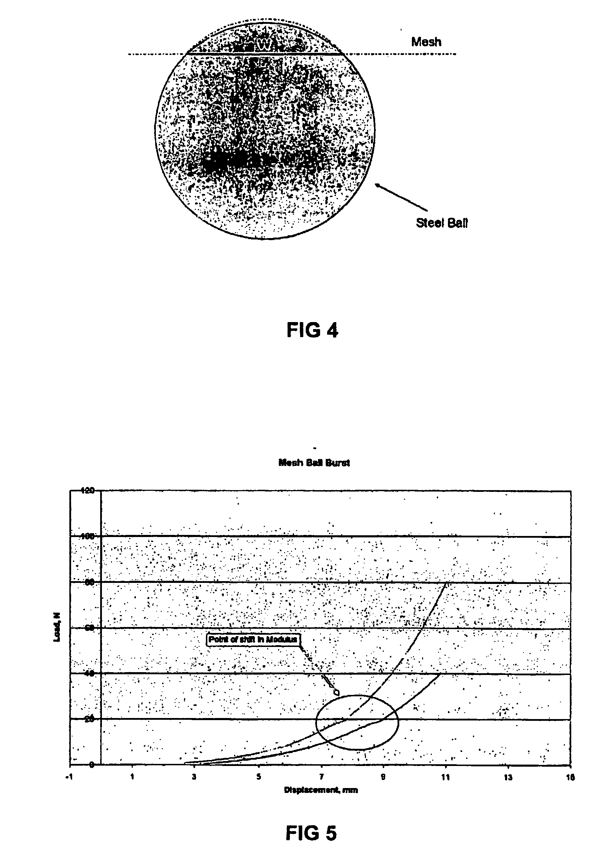

In the BB test a ball is pushed into the mesh and the displacement and load are recorded (see Figure 4). Figure 5 shows Graph 1 which describes BB measurements for three different knit samples as described in Example 5. The experiment was conducted at Room Temperature. The Mesh Tension was calculated using the following formula:

$$\text{Mesh Tension} = \text{Load} / (\pi \times W)$$

Wherein:

W: Width of Steel Ball at the point of displacement
Load: is Load at displacement

Example 8 - Minimum Mesh Tension

The point of shift in modulus is defined as sudden decrease in the slope of the Load vs Displacement curve as depicted in Graph 1 (see Figure 5). The surface tension of the mesh calculated at the point of shift in modulus is defined as Minimum Mesh Tension (hereinafter "MMT"). Minimum mesh tension is calculated as described in Example 7.

As depicted in Graph 1 at point of shift in modulus the value of W is calculated to be 2.33 cm and the load is approximately 18N. Based on this information the average MMT for these samples is calculated to be 2.5 N/cm (MMT = 18N/ (3.14 x 2.33cm) = 2.5N/cm).

**Claims**

1. A knitted article (1) comprising:

 a knitted structure (2) having at least one PTFE fiber (4) with oriented fibrils, said PTFE fiber (4) forming multiple fiber cross-over points (8) wherein said PTFE fiber is self-bonded (10) in at least one of said crossover points (8).

2. The knitted article (10) of claim 1 wherein the fibrils are oriented non-parallel to each other.

3. The knitted article (1) of claim 1 wherein said knitted article (1) is a hernia repair mesh, or wherein said knitted article (1) is a soft tissue repair mesh, or wherein said knitted article (1) is a soft tissue reinforcement mesh, or wherein said knitted article (1) is for repair of anatomical defects and optionally said anatomical defect is abdominal wall, or wherein said knitted article (1) is for correction of defects in the genitourinary system, or wherein said knitted article (1) is for repair of organs.

4. The knitted article (1) of claim 1 further comprising at least one functional layer (14).

5. The knitted article (1) of claim 1 wherein the at least one PTFE fiber (4) is a monofilament PTFE fiber (4).

6. A knitted article (1) according to claim 1, wherein the article (1) is a mesh, said mesh having a mass of between 50 and 70 g/m$^2$ and stiffness of greater than 25g as determined by INDA Standard test Method 90.3; or wherein said mesh has a mass of between 70 and 90 g/m$^2$ and stiffness of greater than 35g as determined by INDA Standard test Method 90.3, or wherein said mesh has a mass of between 90 and 165 g/m$^2$ and stiffness of greater than 50g as determined by INDA Standard test Method 90.3.

7. A knitted article (1) according to claim 1, wherein said PTFE fiber (4) is cross-fibril locked in at least one of said crossover points.

8. A knitted article of claim 1 wherein said PTFE fiber (4) is self-bonded in at least one of said crossover points by heat treatment of said knitted article under tension, and optionally wherein said heat treatment is in the range of 350-375°C.

9. A knitted article according to claim 1, wherein the article (1) is a mesh and wherein said mesh has a mass of greater than 70 g/m$^2$ and stiffness of greater than 25g as determined by INDA Standard test Method 90.3.

10. The knitted article (1) of claim 1 wherein the knitted structure (2) is solely PTFE.

11. The knitted article (1) of claim 1 further comprising at least one additional non-knitted layer.

12. The knitted article (1) of claim 1 further comprising at least one coating covering at least one portion of said knitted structure (2), and optionally wherein the coating is a bioactive agent, or wherein the coating is an antimicrobial agent, or wherein the coating is an antibiotic.

13. A knitted article (1) according to claim 1, wherein the knitted article has a porous structure with an initial stiffness and a second stiffness which is less than the initial stiffness when the porous structure is subjected to Abdominal Mesh Tension.

14. The knitted article (1) of claim 13 wherein the second stiffness is at least 20 % less than the initial stiffness, or wherein the second stiffness is at least 30% less than the initial stiffness, or wherein the second stiffness is at least 40% less than the initial stiffness, or wherein the second stiffness is at least 50% less than the initial stiffness, or wherein the second stiffness is at least 60% less than the initial stiffness, or wherein the Abdominal Mesh Tension is less than 16 N/cm.

15. A method of providing a knitted article, comprising:

 exposing a knitted structure to heat while the knitted structure remains fully constrained, the knitted structure comprising at least one PTFE fiber (4) with oriented fibrils forming multiple fiber cross-over points (8); and heating the knitted structure such that the at least one PTFE fiber (4) becomes self-bonded in at least one of said cross-over points (8).

# EP 2 185 211 B1

**Patentansprüche**

1. Gewirkter Artikel (1), der Folgendes umfasst:

   eine gewirkte Struktur (2), die wenigstens eine PTFE-Faser (4) mit ausgerichteten Fäserchen hat, wobei die PTFE-Faser (4) mehrere Faserkreuzungspunkte (8) bildet, wobei die PTFE-Faser in wenigstens einem der Faserkreuzungspunkte (8) mit sich selbst verbunden (10) ist.

2. Gewirkter Artikel (1) nach Anspruch 1, wobei die Fäserchen nicht-parallel zueinander ausgerichtet sind.

3. Gewirkter Artikel (1) nach Anspruch 1, wobei der gewirkte Artikel (1) ein Bruchverschluss-Netz ist oder wobei der gewirkte Artikel (1) ein Weichgewebe-Reparaturnetz ist oder wobei der gewirkte Artikel (1) ein Weichgewebe-Verstärkungsnetz ist oder wobei der gewirkte Artikel (1) zur Reparatur von anatomischen Defekten dient und wahlweise der anatomische Defekt die Bauchdecke ist oder wobei der gewirkte Artikel (1) zur Korrektur von Defekten im Urogenitalsystem dient oder wobei der gewirkte Artikel (1) zur Reparatur von Organen dient.

4. Gewirkter Artikel (1) nach Anspruch 1, der ferner wenigstens eine funktionelle Lage (14) umfasst.

5. Gewirkter Artikel (1) nach Anspruch 1, wobei die wenigstens eine PTFE-Faser (4) eine Monofilament-PTFE-Faser (4) ist.

6. Gewirkter Artikel (1) nach Anspruch 1, wobei der Artikel (1) ein Netz ist, wobei das Netz eine Masse von zwischen 50 und 70 g/cm$^2$ und eine Steifigkeit von mehr als 25 g, bestimmt durch das INDA-Standard-Prüfverfahren 90.3, hat oder wobei das Netz eine Masse von zwischen 70 und 90 g/cm$^2$ und eine Steifigkeit von mehr als 35 g, bestimmt durch das INDA-Standard-Prüfverfahren 90.3, hat oder wobei das Netz eine Masse von zwischen 90 und 165 g/cm$^2$ und eine Steifigkeit von mehr als 50 g, bestimmt durch das INDA-Standard-Prüfverfahren 90.3, hat.

7. Gewirkter Artikel (1) nach Anspruch 1, wobei die PTFE-Faser (4) durch Kreuzen der Fäserchen in wenigstens einem der Kreuzungspunkte blockiert ist.

8. Gewirkter Artikel nach Anspruch 1, wobei die PTFE-Faser (4) durch eine Wärmebehandlung des gewirkten Artikels unter Spannung in wenigstens einem der Kreuzungspunkte mit sich selbst verbunden ist und wobei wahlweise die Wärmebehandlung in dem Bereich von 350 bis 375°C erfolgt.

9. Gewirkter Artikel nach Anspruch 1, wobei der Artikel (1) ein Netz ist und wobei das Netz eine Masse von mehr als 70 g/cm$^2$ und eine Steifigkeit von mehr als 25 g, bestimmt durch das INDA-Standard-Prüfverfahren 90.3, hat.

10. Gewirkter Artikel (1) nach Anspruch 1, wobei die gewirkte Struktur (2) nur aus PTFE besteht.

11. Gewirkter Artikel (1) nach Anspruch 1, der ferner wenigstens eine zusätzliche nicht gewirkte Lage umfasst.

12. Gewirkter Artikel (1) nach Anspruch 1, der ferner wenigstens eine Beschichtung umfasst, die wenigstens einen Abschnitt der gewirkten Struktur (2) bedeckt, und wobei wahlweise die Beschichtung ein bioaktiver Wirkstoff ist oder wobei die Beschichtung ein antimikrobieller Wirkstoff ist oder wobei die Beschichtung ein Antibiotikum ist.

13. Gewirkter Artikel (1) nach Anspruch 1, wobei der gewirkte Artikel eine poröse Struktur mit einer anfänglichen Steifigkeit und einer zweiten Steifigkeit, die geringer ist als die anfängliche Steifigkeit, wenn die poröse Struktur einer Bauchnetzspannung ausgesetzt wird.

14. Gewirkter Artikel (1) nach Anspruch 13, wobei die zweite Steifigkeit wenigstens 20 % geringer ist als die anfängliche Steifigkeit oder wobei die zweite Steifigkeit wenigstens 30 % geringer ist als die anfängliche Steifigkeit oder wobei die zweite Steifigkeit wenigstens 40 % geringer ist als die anfängliche Steifigkeit oder wobei die zweite Steifigkeit wenigstens 50 % geringer ist als die anfängliche Steifigkeit oder wobei die zweite Steifigkeit wenigstens 60 % geringer ist als die anfängliche Steifigkeit oder wobei die Bauchnetzspannung geringer ist als 16 N/cm.

15. Verfahren zum Bereitstellen eines gewirkten Artikels, das Folgendes umfasst:

   das Behandeln einer gewirkten Struktur mit Wärme, während die gewirkte Struktur vollständig eingespannt bleibt, wobei die gewirkte Struktur wenigstens eine PTFE-Faser (4) mit ausgerichteten Fäserchen umfasst, die mehrere Faserkreuzungspunkte (8) bilden, und

das Erhitzen der gewirkten Struktur derart, dass die wenigstens eine PTFE-Faser (4) in wenigstens einem der Faserkreuzungspunkte (8) mit sich selbst verbunden wird.

**Revendications**

1. Article tricoté (1), comprenant :

   une structure tricotée (2) comportant au moins une fibre de PTFE (4) avec des fibrilles orientées, ladite fibre de PTFE (4) formant de multiples points de croisement de fibres (8), ladite fibre de PTFE étant auto-liée (10) dans au moins un desdits points de croisement (8).

2. Article tricoté (1) selon la revendication 1, dans lequel les fibrilles sont orientées de manière non parallèle les unes par rapport aux autres.

3. Article tricoté (1) selon la revendication 1, dans lequel ledit article tricoté (1) est une maille de réparation de la hernie, ou dans lequel ledit article tricoté (1) est une maille de réparation des tissus mous, ou dans lequel ledit article tricoté (1) est une maille de renforcement des tissu mous, ou dans lequel ledit article tricoté (1) sert à la réparation de défauts anatomiques, ledit défaut anatomique étant optionnellement constitué par la paroi abdominale, ou dans lequel ledit article tricoté (1) sert à corriger des défauts dans le système génito-urinaire, ou dans lequel ledit article tricoté (1) sert à la réparation d'organes.

4. Article tricoté (1) selon la revendication 1, comprenant en outre au moins une couche fonctionnelle (14).

5. Article tricoté (1) selon la revendication 1, dans lequel la au moins une fibre de PTFE (4) est une fibre de PTFE monofilament (4).

6. Article tricoté (1) selon la revendication 1, dans lequel l'article (1) est une maille, ladite maille ayant une masse comprise entre 50 et 70 g/m$^2$ et une rigidité supérieure à 25g, comme déterminé par la méthode d'essai selon la norme INDA 90.3 ; ou dans lequel ladite maille a une masse comprise entre 70 et 90 g/m$^2$ et une rigidité supérieure à 35g, comme déterminé par la méthode d'essai selon la norme INDA 90.3, ou dans lequel ladite maille a une masse comprise entre 90 et 165 g/m$^2$ et une rigidité supérieure à 50g, comme déterminé par la méthode d'essai selon la norme INDA 90.3.

7. Article tricoté (1) selon la revendication 1, dans lequel ladite fibre de PTFE (4) est bloquée par croisement de fibrilles dans au moins un desdits points de croisement.

8. Article tricoté selon la revendication 1, dans lequel ladite fibre de PTFE est auto-liée dans au moins un desdits points de croisement par un traitement thermique dudit article tricoté sous tension, ledit traitement thermique étant optionnellement effectué dans la plage de températures allant de 350 à 375°C.

9. Article tricoté selon la revendication 1, dans lequel l'article (1) est une maille, ladite maille ayant une masse supérieure à 70g/m$^2$ et une rigidité supérieure à 25g, comme déterminé par la méthode d'essai selon la norme INDA 90.3.

10. Article tricoté (1) selon la revendication 1, dans lequel la structure tricotée (2) est uniquement composée de PTFE.

11. Article tricoté (1) selon la revendication 1, comprenant en outre au moins une couche additionnelle non tricotée.

12. Article tricoté (1) selon la revendication 1, comprenant en outre au moins un revêtement recouvrant au moins une partie de ladite structure tricotée (2), ledit revêtement étant optionnellement constitué par un agent bioactif, ou dans lequel le revêtement est constitué par un agent antimicrobien, ou dans lequel le revêtement est constitué par un antibiotique.

13. Article tricoté (1) selon la revendication 1, dans lequel l'article tricoté a une structure poreuse, avec une rigidité initiale et une deuxième rigidité, inférieure à la rigidité initiale, lorsque la structure poreuse est soumise à une tension de la maille abdominale.

**14.** Article tricoté selon la revendication 13, dans lequel la deuxième rigidité est inférieure d'au moins 20 % à la rigidité initiale, ou dans lequel la deuxième rigidité est inférieure d'au moins 30 % à la rigidité initiale ou dans lequel la deuxième rigidité est inférieure d'au moins 40 % à la rigidité initiale, ou dans lequel la deuxième rigidité est inférieure d'au moins 50% à la rigidité initiale, ou dans lequel la deuxième rigidité est inférieure d'au moins 60% à la rigidité initiale, ou dans lequel la tension de la maille abdominale est inférieure à 16 N/cm.

**15.** Procédé de fourniture d'un article tricoté, comprenant les étapes ci-dessous :

exposition d'une structure tricotée à la chaleur, pendant que la structure tricoté reste complètement sous contrainte, la structure tricotée comprenant au moins une fibre de PTFE (4) avec des fibrilles orientées formant de multiples points de croisement des fibres (8) ; et
chauffage de la structure tricotée, de sorte que la au moins une fibre de PTFE (4) est auto-liée dans au moins un desdits points de croisement (8).

FIG. 1A

FIG. 1B

FIG. 1C

FIG. 1D

FIG. 2

FIG. 3A

FIG. 3B

FIG. 3C

FIG. 3D

FIG. 3E

EP 2 185 211 B1

Mesh

Steel Ball

FIG 4

Mesh Ball Burst

Point of shift in Modulus

Load, N

Displacement, mm

FIG 5

15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050222591 A, Gingras

**Non-patent literature cited in the description**

- US Markets for Soft Tissue Repair. Millennium Research Group, 2006
- European Markets for Soft Tissue Repair. Millennium Research Group, 2006
- **KLOSTERHALFEN et al.** *Expert Rev Med Devices,* January 2005, vol. 2 (1), 103-17
- **KLINGE et al.** *Eur J Surg,* 1999, vol. 165, 665-673
- **COBB et al.** Normal Intraabdominal Pressure in Healthy Adults. *J Surg Res.,* 02 September 2005, vol. 129 (2), 231-5
- **KLINGE et al.** Modified mesh for hernia repair that is adapted to the Physiology of the abdominal wall. *EurJ Surg.,* December 1998, vol. 164 (12), 951-60